# EUROPEAN PATENT APPLICATION

(11) **EP 4 083 892 A1**
(43) Date of publication of application: **02.11.2022**
(21) Application number: 22169428.4
(22) Date of filing: 22.04.2022
(51) Int. Cl.: G06Q 30/02, G06Q 10/06, G06Q 10/00

(54) **REGISTRATION SYSTEM AND REGISTRATION METHOD**

(30) Priority: 29.04.2021 TW 110115628
(71) Applicant: AI Bioelectronic Healthtech Co., Ltd., 32450 Taoyuan City (TW)
(72) Inventor: HO, Yen-Yi, 32450 Taoyuan City (TW)
(74) Representative: Viering, Jentschura & Partner mbB Patent- und Rechtsanwälte

(57) **Abstract**

A registration method is applied to a registration system (1), wherein the registration system (1) includes a registration request receiving module (10), a period determining module (20) and a registration fee calculation module (40). The registration method includes: via the registration request receiving module (10), receiving a registration data, and the registration data includes a registration time and a registration request; via the period determining module (20), determining if the registration time is in a peak hour according to the registration time; if the registration time is in a peak hour, then via the registration fee calculation module (40), calculating a peak registration fee according to the registration time and the registration request; if the registration time is not in a peak hour, then via the registration fee calculation module (40), calculating a normal registration fee according to the registration time and the registration request.

## Description

### BACKGROUND OF THE INVENTION

### 1. Field of the Invention

The present invention relates to a registration system and registration method; more particularly, the present invention relates to a registration system and registration method providing different charging mechanisms to achieve the function of reducing the patients' overall waiting time.

### 2. Description of the Related Art

When people develop an illness, they will go to a hospital or clinic to register and queue up to see a doctor according to a registration number. General hospitals and clinics have peak hours for registration, such as the evening hours from Monday to Friday (that is, after general office workers finish their normal working hours). In peak hours, many patients may want to register to see a doctor, so a patient may have to spend a lot of time waiting in a queue. Similarly, hospitals and clinics also have off-peak hours, such as from 8 am to 6 pm from Monday to Friday (that is, the normal working hours for general office workers). In off-peak hours, fewer patients want to register to see the doctor, so the patients in off-peak hours do not need to spend too much time waiting in a queue.

Therefore, there is a need for a method to divert patients from peak hours to off-peak hours for registration so as to reduce patients' overall waiting time.

### SUMMARY OF THE INVENTION

The object of the present invention is to provide a registration method which achieves the function of reducing the patients' overall waiting time by providing different charging mechanisms.

To achieve the abovementioned object, the registration method of the present invention is applied to a registration system, and the registration system includes a registration request receiving module, a period determining module and a registration fee calculation module. The registration method includes: via the registration request receiving module, receiving a registration data, wherein the registration data includes a registration time and a registration request; via the period determining module, determining if the registration time is in a peak hour according to the registration time; if the registration time is in a peak hour, then via the registration fee calculation module, calculating a peak registration fee according to the registration time and the registration request; if the registration time is not in a peak hour, then via the registration fee calculation module, calculating a normal registration fee according to the registration time and the registration request.

According to one embodiment of the present invention, the registration system further includes a charge recording module and a registration serial number controlling module. The registration method further includes: via the charge recording module, receiving a charge fee data; via the registration serial number controlling module, determining if the charge fee data includes the expedited registration fee; if so, then via the registration serial number controlling module, providing a quick queuing serial number; if not, then via the registration serial number controlling module, providing a normal queuing serial number.

According to one embodiment of the present invention, the registration system further includes a registered people calculation module. The registration method further includes: via the registered people calculation module, calculating a number of people queuing.

According to one embodiment of the present invention, the step in which, if the registration time is in a peak hour, then via the registration fee calculation module, calculating the peak registration fee according to the registration time and the registration request further includes: if the registration time is in a peak hour and the number of people queuing is more than a default value, then via the registration fee calculation module, calculating the peak registration fee according to the registration time, the registration request and the number of people queuing. The step in which, if the registration time is not in a peak hour, then via the registration fee calculation module, calculating the normal registration fee according to the registration time and the registration request further includes: if the registration time is not in a peak hour and the number of people queuing is less than the default value, then via the registration fee calculation module, calculating the normal registration fee according to the registration time, the registration request and the number of people queuing.

According to one embodiment of the present invention, the registration system further includes an insurance and cash flow module. The registration method further includes: via the insurance and cash flow module, generating an insurance and cash flow data according to the charge fee data.

According to one embodiment of the present invention, a range of the peak hours is between 6 pm and 10 pm.

Another object of the present invention is to provide a registration system which achieves the function of reducing the patients' overall waiting time by providing different charging mechanisms.

To achieve the abovementioned object, a registration system of the present invention includes a registration request receiving module, a period determining module and a registration fee calculation module. The registration request receiving module is used for receiving a registration data, wherein the registration data includes a registration time and a registration request. The period determining module is used for determining if the registration time is in a peak hour according to the registration time. The registration fee calculation module is electrically connected to the registration request receiving module and the period determining module. If the registration time is in a peak hour, the registration fee calculation module will calculate a peak registration fee according to the registration time and the registration request; if the registration time is not in a peak hour, the registration fee calculation module will calculate a normal registration fee according to the registration time and the registration request.

According to one embodiment of the present invention, the registration fee calculation module is further used for calculating an expedited registration fee according to a registration request. The registration system further includes a charge recording module and a registration serial number controlling module. The charge recording module is used for receiving a charge fee data. The registration serial number controlling module is electrically connected to the registration fee calculation module and the charge recording module. The registration serial number controlling module is used for determining if the charge fee data includes the expedited registration fee; if so, the registration serial number controlling module will provide a quick queuing serial number; if not, the registration serial number controlling module will provide a normal queuing serial number.

According to one embodiment of the present invention, the registration system further includes a registered people calculation module, the registered people calculation module is electrically connected to the registration fee calculation module, and the registered people calculation module is used for calculating a number of people queuing.

According to one embodiment of the present invention, if the registration time is in a peak hour and the number of people queuing is equal to or higher than a default value, the registration fee calculation module will calculate the peak registration fee according to the registration time, the registration request and the number of people queuing; if the registration time is not in a peak hour and the number of people queuing is less than the default value, the registration fee calculation module will calculate the normal registration fee according to the registration time, the registration request and the number of people queuing.

According to one embodiment of the present invention, the registration system further includes an insurance and cash flow module, the insurance and cash flow module is electrically connected to the charge recording module, and the insurance and cash flow module is used for generating an insurance and cash flow data according to the charge fee data.

### BRIEF DESCRIPTION OF THE DRAWINGS

FIG. 1 illustrates a system structure drawing of the registration system in one embodiment of the present invention.
FIG. 2 illustrates a step flowchart of the registration method in one embodiment of the present invention.

### DETAILED DESCRIPTION OF THE PREFERRED EMBODIMENT

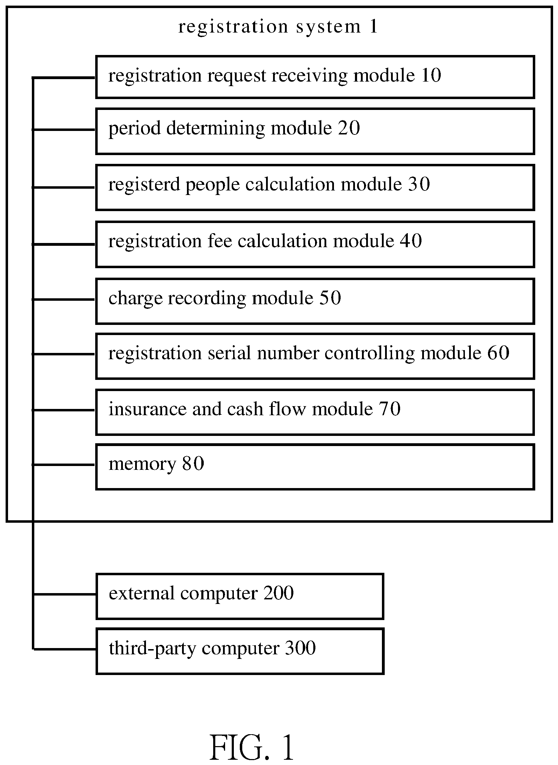

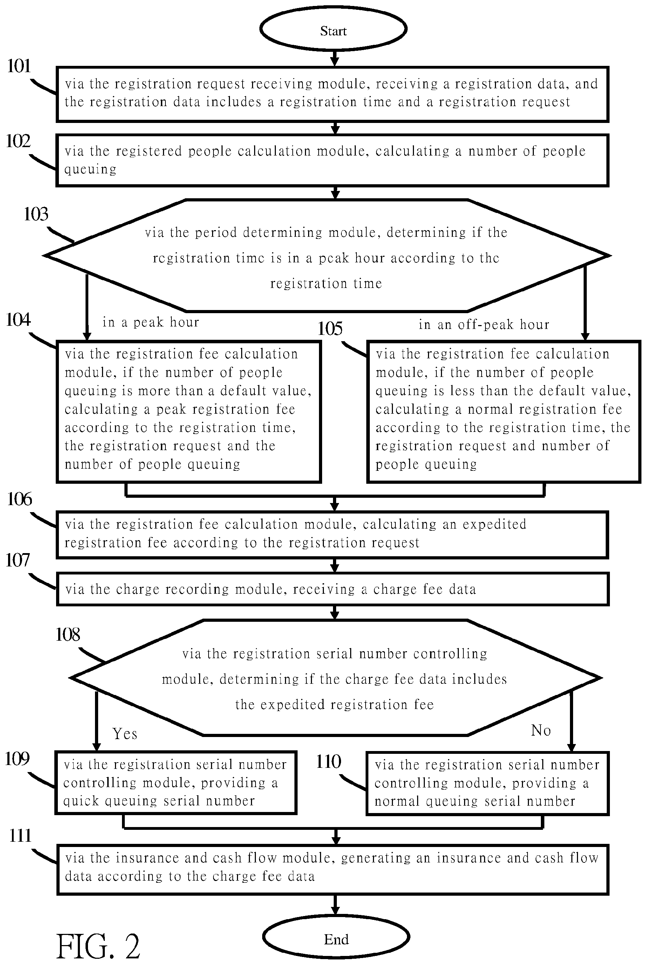

Please refer to FIG. 1 to FIG. 2, which illustrate the registration system and the registration method in one embodiment of the present invention. FIG. 1 illustrates a system structure drawing of the registration system in one embodiment of the present invention. FIG. 2 illustrates a step flowchart of the registration method in one embodiment of the present invention.

As shown in FIG. 1 and FIG. 2, in one embodiment of the present invention, the registration system 1 and the registration method provide different charging mechanisms to distribute the patients of the peak hours to the off-peak hours for registration so as to achieve the function of reducing the patients' overall waiting time. The registration system 1 is a server installed in a hospital or a clinic and electrically connected to an external computer 200 or a third-party computer 300 (such as the computer of an insurance company or a financial institution) on the network. The registration system 1 includes a registration request receiving module 10, a period determining module 20, a registered people calculation module 30, a registration fee calculation module 40, a charge recording module 50, a registration serial number controlling module 60, an insurance and cash flow module 70 and a memory 80.

In one embodiment of the present invention, the registration request receiving module 10 is a network card for receiving a registration data sent from the external computer 200 at a registration time, and the registration data includes the registration time and a registration request. The registration time is the time when the patient sees a doctor for medical treatment. The registration request includes the identity information of the patient and the outpatient clinic where the patient wants to register. The period determining module 20 is a chip with a time determining function for determining if the registration time is in a peak hour according to the registration time. The range of the peak hours is between 6 pm and 10 pm, but the range of the peak hours is not limited to that design, for it can be changed according to actual requirements.

The registered people calculation module 30 is a chip with a people amount calculating function for calculating a default value of the number of people queuing in advance and calculating the number of people who are currently queuing for registration in any outpatient department. The registration fee calculation module 40 is a chip with a function of calculating a registration fee for calculating a registration fee and an expedited registration fee according to the registration time, the registration request and the number of people queuing. If the registration time is in a peak hour and the number of people queuing is more than a default value, the registration fee calculation module 40 will calculate a peak registration fee according to the registration time, the registration request and the number of people queuing. If the registration time is not in a peak hour and the number of people queuing is less than the default value, the registration fee calculation module 40 will calculate the normal registration fee according to the registration time, the registration request and the number of people queuing, wherein the peak registration fee is higher than the normal registration fee.

The charge recording module 50 is a chip with a data recording and integration function for receiving a charge fee data. The charge fee data is the related data of the registration fee paid by the patient sent from the external computer 200, and the charge fee data includes a peak registration fee, a normal registration fee, or an expedited registration fee according to the registration data of the patient. The registration serial number controlling module 60 is used for determining if the charge fee data includes an expedited registration fee. If the charge fee data includes an expedited registration fee, the registration serial number controlling module 60 will provide a quick queuing serial number. If the charge fee data does not include an expedited registration fee, the registration serial number controlling module 60 will provide a normal queuing serial number, and the queuing order of the quick queuing serial number is lower than the normal queuing serial number.

The insurance and cash flow module 70 is a chip for processing and generating the insurance and cash flow data for generating insurance and cash flow data and sending the insurance and cash flow data to a third-party computer 300 according to the charge fee data. The memory 80 is electrically connected to the registration request receiving module 10, the period determining module 20, the registered people calculation module 30, the registration fee calculation module 40, the charge recording module 50, the registration serial number controlling module 60 and the insurance and cash flow module 70. The memory 80 is used for storing the data and the software for executing the registration method.

The registration method of the present invention is programmed as a software and stored in the memory 80. When a patient goes to a hospital or clinic to see a doctor, the patient must first go to the counter and ask to register, whereupon the counter service staff will operate the external computer 200 to access to the registration system 1 to send a registration data; at this moment, the registration system 1 executes step 101 of the registration method: via the registration request receiving module, receiving a registration data, and the registration data includes a registration time and a registration request.

The registration request receiving module 10 receives the registration data sent by the external computer 200, and the registration data includes a registration time and a registration request. The registration time is the time when the patient will see a doctor for medical treatment. The registration request includes the identity information of the patient and the outpatient clinic where the patient wants to register.

Then the registration system 1 executes step 102 of the registration method: via the registered people calculation module, calculating a number of people queuing.

After the registration request receiving module 10 sends the registration data to the registered people calculation module 30 and each patient's outpatient service is completed, the completed outpatient data will be sent to the registered people calculation module 30 so that the registered people calculation module 30 can calculate the current receiving registration data and the number of patients whose outpatient service is completed so as to calculate the current number of people queuing.

Then the registration system 1 executes step 103 of the registration method: via the period determining module, determining if the registration time is in a peak hour according to the registration time.

The registration request receiving module 10 sends the receiving registration data to the period determining module 20; the period determining module 20 determines if the registration time is in a peak hour according to the registration time of the registration data; the range of the peak hours of the present invention is between 6 pm and 10 pm. If the period determining module 20 determines that the registration time is in a peak hour, the registration system 1 will execute step 104 of the registration method: via the registration fee calculation module, if the number of people queuing is more than a default value, calculating a peak registration fee according to the registration time, the registration request and the number of people queuing.

The registration request receiving module 10 sends the receiving registration data to the registration fee calculation module 40; then the period determining module 20 sends the information that the registration time is in a peak hour to the registration fee calculation module 40; the registered people calculation module 30 also sends the current number of people queuing and the default value to the registration fee calculation module 40 so that the registration fee calculation module 40 can calculate the peak registration fee according to the registration time of the peak hour, the registration request and the number of people queuing. For example, if the number of people queuing is more than the default value (wherein the default value is ten people), the registration fee calculation module 40 will calculate a higher peak registration fee (such as 300 dollars) to discourage patients from registering during the peak hour. However, if the number of people queuing is not more than the default value and there is no need to discourage patients from registering during the peak hour, the registration fee calculation module 40 will calculate a lower peak registration fee (such as 200 dollars).

Please return to step 103; if the period determining module 20 determines that the registration time is not in a peak hour, the registration system 1 will execute step 105 of the registration method: via the registration fee calculation module, if the number of people queuing is less than the default value, calculating a normal registration fee according to the registration time, the registration request and the number of people queuing.

The registration request receiving module 10 sends the receiving registration data to the registration fee calculation module 40, and the period determining module 20 sends the information that the registration time is not in a peak hour to the registration fee calculation module 40. The registered people calculation module 30 also sends the current number of people queuing and the default value to the registration fee calculation module 40 so that the registration fee calculation module 40 can calculate the normal registration fee according to the registration time of off-peak hours, the registration request and the number of people queuing; for example, if the number of people queuing is more than the default value (wherein the default value is ten people), then the registration fee calculation module 40 will calculate a higher normal registration fee (such as 150 dollars) to discourage patients from registering during the peak hour; however, if the number of people queuing is not more than the default value and there is no need to discourage patients from registering during the peak hour, the registration fee calculation module 40 will calculate a lower normal registration fee (such as 100 dollars). It is to be known that the normal registration fee calculated by the registration fee calculation module 40 is lower than the peak registration fee to encourage patients to register in off-peak hours so as to divert registrations to off-peak hours and thereby reduce the overall waiting time.

After the registration fee calculation module 40 calculates the normal registration fee or the peak registration fee, the registration system 1 will execute step 106 of the registration method: via the registration fee calculation module, calculating an expedited registration fee according to the registration request.

The registration fee calculation module 40 checks the clinic where the patient wants to register according to the registration request to calculate an expedited registration fee (such as 100 dollars). The expedited registration fee is designed to allow a patient to choose to pay an extra fee to move the patient forward in the registration queue so as to reduce the queuing time of the patient who pays the extra fee. Because the queuing time of the patient is reduced, the queuing time of other patients is relatively increased; thus, a portion of the expedited registration fee paid by the patient can be provided to other patients as compensation, and the remaining portion can be provided to the hospital or the clinic as additional income.

After the registration fee calculation module 40 calculates the expedited registration fee, the peak registration fee or the normal registration fee, the counter staff can inform the patient of the abovementioned fees so that the patient can decide whether he/she needs to pay an extra expedited registration fee in addition to the registration fee. Then the registration system 1 executes step 107 of the registration method: via the charge recording module, receiving a charge fee data.

After the patient knows the details of each fee, the patient can pay the counter staff according to the requirement. After the counter staff receives the fee, the counter staff can send the details of the receiving fee to the charge recording module 50 of the registration system 1 via the external computer 200.

Then the registration system 1 executes step 108 of the registration method: via the registration serial number controlling module, determining if the charge fee data includes the expedited registration fee.

The charge recording module 50 sends the charge fee data to the registration serial number controlling module 60, and the registration serial number controlling module 60 checks the details of the charge fee data to determine if the charge fee data includes the expedited registration fee. If the registration serial number controlling module 60 determines that the charge fee data includes the expedited registration fee, the registration system 1 will execute step 109 of the registration method: via the registration serial number controlling module, providing a quick queuing serial number.

After the registration serial number controlling module 60 determines that the charge fee data includes the expedited registration fee, the registration serial number controlling module 60 will provide a quick queuing serial number to the external computer 900 so that the counter staff can tell the patient about the quick queuing serial number and the patient can wait to see the doctor according to the quick queuing serial number.

Please return to step 108; if the registration serial number controlling module 60 determines that the charge fee data does not include the expedited registration fee, the registration system 1 will execute step 110 of the registration method: via the registration serial number controlling module, providing a normal queuing serial number.

After the registration serial number controlling module 60 determines that the charge fee data does not include the expedited registration fee, the registration serial number controlling module 60 will provide a normal queuing serial number to the external computer 900 so that the counter staff can tell the patient about the normal queuing serial number and the patient can wait to see the doctor according to the normal queuing serial number, and the quick queuing serial number is lower than the normal queuing serial number; therefore, a patient who pays the expedited registration fee can queue up to see a doctor faster.

Finally, the registration system 1 executes step 111 of the registration method: via the insurance and cash flow module, generating an insurance and cash flow data according to the charge fee data.

The charge recording module 50 sends the charge fee data to the insurance and cash flow module 70. The insurance and cash flow module 70 generates an insurance and cash flow data according to the charge fee data and sends the insurance and cash flow data to the insurance company or financial institution of the third-party computer 300 so that the insurance company or financial institution of the third-party computer 300 can use the insurance and cash flow data to perform the insurance and cash flow process for the patient.

Via the registration system 1 and the registration method of the present invention, different registration fees can be provided for peak hours and off-peak hours to divert patients from peak hours to off-peak hours so as to reduce the patients' overall waiting time. Furthermore, the expedited registration fee option can be provided to the patient so that the patient can choose to pay an extra fee so as to move forward in the registration queue and thereby reduce the queuing time of a patient who pays the extra fee, and a portion of the expedited registration fee paid by the patient can be provided to other patients as compensation, and the remaining portion can be provided to the hospital or the clinic as additional income.

## Claims

1. A registration method, applied to a registration system (1), wherein the registration system (1) comprises a registration request receiving module (10), a period determining module (20) and a registration fee calculation module (40), the registration method comprising: via the registration request receiving module (10), receiving a registration data, wherein the registration data comprises a registration time and a registration request; via the period determining module (20), according to the registration time, determining if the registration time is in a peak hour; if the registration time is in a peak hour, then via the registration fee calculation module (40), calculating a peak registration fee according to the registration time and the registration request; and if the registration time is not in a peak hour, then via the registration fee calculation module (40), calculating a normal registration fee according to the registration time and the registration request.

2. The registration method as claimed in Claim 1, wherein the registration system (1) further comprises a charge recording module (50) and a registration serial number controlling module (60), and the registration method further comprises: via the charge recording module (50), receiving a charge fee data; via the registration serial number controlling module (60), determining if the charge fee data comprises an expedited registration fee; if so, then via the registration serial number controlling module (60), providing a quick queuing serial number; and if not, then via the registration serial number controlling module (60), providing a normal queuing serial number.

3. The registration method as claimed in Claim 2, wherein the registration system (1) further comprises a registered people calculation module (30), and the registration method further comprises:
via the registered people calculation module (30), calculating a number of people queuing.

4. The registration method as claimed in Claim 3, wherein the step in which if the registration time is in a peak hour, then via the registration fee calculation module (40), calculating a peak registration fee according to the registration time and the registration request further comprises:
if the registration time is in a peak hour and the number of people queuing is more than a default value, then via the registration fee calculation module (40), calculating the peak registration fee according to the registration time, the registration request and the number of people queuing;
wherein the step in which if the registration time is not in a peak hour, then via the registration fee calculation module (40), calculating a normal registration fee according to the registration time and the registration request further comprises: if the registration time is not in a peak hour and the number of people queuing is less than the default value, then via the registration fee calculation module (40), calculating the normal registration fee according to the registration time, the registration request and the number of people queuing.

5. The registration method as claimed in Claim 4, wherein the registration system (1) further comprises an insurance and cash flow module (70), and the registration method further comprises: via the insurance and cash flow module (70), generating an insurance and cash flow data according to the charge fee data.

6. The registration method as claimed in Claim 4, wherein a range of the peak hours is between 6 pm and 10 pm.

7. A registration system (1), comprising:
a registration request receiving module (10) for receiving a registration data, wherein the registration data comprises a registration time and a registration request;
a period determining module (20) for determining if the registration time is in a peak hour according to the registration time; and a registration fee calculation module (40), electrically connected to the registration request receiving module (10) and the period determining module (20);
wherein if the registration time is in a peak hour, the registration fee calculation module (40) will calculate a peak registration fee according to the registration time and the registration request; if the registration time is not in a peak hour, the registration fee calculation module (40) will calculate a normal registration fee according to the registration time and the registration request.

8. The registration system (1) as claimed in Claim 7, wherein the registration fee calculation module (40) is further used for calculating an expedited registration fee according to the registration request; the registration system (1) further comprises a charge recording module (50) and a registration serial number controlling module (60), and the charge recording module (50) is used for receiving a charge fee data; the registration serial number controlling module (60) is electrically connected to the registration fee calculation module (40) and the charge recording module (50), and the registration serial number controlling module (60) is used for determining if the charge fee data comprises the expedited registration fee; if so, the registration serial number controlling module (60) provides a quick queuing serial number; if not, the registration serial number controlling module (60) provides a normal queuing serial number.

9. The registration system (1) as claimed in Claim 8, further comprising a registered people calculation module (30), wherein the registered people calculation module (30) is electrically connected to the registration fee calculation module (40), and the registered people calculation module (30) is used for calculating a number of people queuing.

10. The registration system (1) as claimed in Claim 9, wherein if the registration time is in a peak hour and the number of people queuing is more than a default value, the registration fee calculation module (40) will calculate the peak registration fee according to the registration time, the registration request and the number of people queuing; if the registration time is not in a peak hour and the number of people queuing is less than the default value, the registration fee calculation module (40) will calculate the normal registration fee according to the registration time, the registration request and the number of people queuing.

11. The registration system (1) as claimed in Claim 10, further comprising an insurance and cash flow module (70), wherein the insurance and cash flow module (70) is electrically connected to the charge recording module (50), and the insurance and cash flow module (70) is used for generating an insurance and cash flow data according to the charge fee data.

12. The registration system (1) as claimed in Claim 11, wherein a range of the peak hours is between 6 pm and 10 pm.
